# EUROPEAN PATENT SPECIFICATION

(11) **EP 3 910 067 B1**
(45) Date of publication and mention of the grant of the patent: **09.08.2023**
(21) Application number: 19908402.1
(22) Date of filing: 29.04.2019
(51) Int. Cl.: C12Q 1/68, C12Q 1/6844

(54) **NUCLEIC ACID RELEASE AGENT, NUCLEIC ACID PCR AMPLIFICATION METHOD AND PCR AMPLIFICATION KIT**
NUKLEINSÄURETRENNMITTEL, NUKLEINSÄURE-PCR-AMPLIFIKATIONSVERFAHREN UND PCR-AMPLIFIKATIONSKIT
AGENT DE LIBÉRATION D'ACIDE NUCLÉIQUE, PROCÉDÉ D'AMPLIFICATION PCR D'ACIDE NUCLÉIQUE ET KIT D'AMPLIFICATION PCR

(30) Priority: 08.01.2019 CN 201910014324
(43) Date of publication of application: 17.11.2021
(73) Proprietor: Sansure Biotech Inc., Changsha, Hunan 410205 (CN)
(72) Inventor: DAI, Lizhong, Changsha, Hunan 410205 (CN); JI, Bozhi, Changsha, Hunan 410205 (CN); ZHANG, Wenqu, Changsha, Hunan 410205 (CN); DENG, Zhongping, Changsha, Hunan 410205 (CN); FAN, Wenzhou, Changsha, Hunan 410205 (CN)
(74) Representative: Grünecker Patent- und Rechtsanwälte PartG mbB
(86) International application number: PCT/CN2019/085066
(87) International publication number: WO 2020/143135

(56) References cited:
- WO-A1-94/26867
- WO-A1-2007/146197
- CN-A- 105 734 044
- CN-A- 107 022 651
- CN-A- 109 402 240
- Saeed El-Ashram ,ibrahim Al Nasr , Xun Suo: "Nucleic acid protocols:extraction and optimization", Biotechnology Reports, vol. 12, 31 December 2016 (2016-12-31), pages 33-39, XP055718867, DOI: 10.1016/j.btre.2016.10.001

## Description

### TECHNICAL FIELD

The present disclosure relates to the field of molecular biology, in particular to a nucleic acid release agent, a method for PCR amplification of a nucleic acid and a PCR amplification kit.

### BACKGROUND

Polymerase chain reaction (PCR) is a molecular biological technique for amplifying a specific nucleic acid fragment, importantly characterized by massive enrichment and increase of a trace amount of nucleic acid, so as to facilitate the detection of the trace amount of nucleic acid. The commonly used PCR method in medical diagnosis is mainly a real-time fluorescent quantitative PCR (qPCR) method based on dual fluorescent probes. Targets for in vitro diagnosis using the qPCR method mainly include human genomic DNA, DNA viruses, bacteria, fungi, RNA viruses, and the like. WO 94/26867 A1 relates to a process to disrupt virions and isolate the nucleic acids of a virus by provision of a direct lysis buffer comprising a combination of Proteinase K and detergents selected from sodium dodecyl sulfate, Triton X-100 and Tween 20. WO 2007/146197 A1 relates to methods and reagents for virus isolation and detection, in particular to a viral-particle isolation reagent, comprising a virus-particle binding agent and a solid support, such as an HIV or Hepatitis virus capture reagent. However, since RNA has a single-stranded structure, which is unstable and easily degraded, a process of sample treatment is subjected to very stringent requirements, and a more complicated method is required for pretreatment, nucleic acid extraction and purification of the RNA sample to be amplified to obtain the pure nucleic acid, then the detection can be performed to obtain a stable result.

### SUMMARY

Accordingly, it is necessary to provide a nucleic acid release agent that can simplify the PCR amplification method of RNA samples.

The present disclosure provides a nucleic acid release agent comprising Tris-HCl, sodium chloride, potassium chloride, Tween 20, Triton X-100, ethyl phenyl polyethylene glycol and a strong base; wherein Tris-HCl has a molar concentration ranging from 0.5 mM to 500 mM, sodium chloride has a molar concentration from 20 mM to 500 mM, potassium chloride has a mass concentration ranging from 5 mg/mL to 8 mg/mL, Tween 20 has a volume percentage ranging from 0.1% to 2%, Triton X-100 has a volume percentage ranging from 0.1% to 3%, ethyl phenyl polyethylene glycol has a volume percentage ranging from 0.1% to 3%, and the strong base has a mass concentration ranging from 2 mg/mL to 50 mg/mL.

In the nucleic acid release agent of the present disclosure, a certain proportion of the strong base lyses cells, which release nucleic acids. Sodium chloride and potassium chloride protect nucleic acids by coordinating the balance of intracellular and extracellular ions. Tris-HCl is for keeping a pH value stable during cell lysis and better compatible with a PCR reaction solution in subsequent amplification. Triton X-100, on the one hand, can protect nucleic acids, especially single-stranded RNA, allowing RNAto be stored in an alkaline environment. On the other hand, Triton X-100 with potassium chloride in a certain ratio can reduce the inhibitory effect of the strong alkaline environment on the enzyme in the PCR reaction, thereby ensuring the amplification efficiency of RNA. Tween 20 and ethyl phenyl polyethylene glycol can protect reverse transcriptase, so that the reverse transcriptase works normally in the alkaline environment. Through the synergy of the above components, the nucleic acid release agent of the present disclosure enables a sample containing RNA to directly release RNA at room temperature, thereby avoiding the problem of sample contamination caused by aerosol generated by heating, and effectively preventing RNA from degrading in the alkaline environment. More importantly, it can be directly mixed with the PCR reaction solution for PCR amplification to complete the amplification without the need for complicated nucleic acid extraction and purification processes. This truly realizes a one-chamber, pollution-free, simple and rapid RNA amplification and detection, with high detection sensitivity and good repeatability.

In one of the embodiments, the nucleic acid release agent further comprises betaine and bovine serum albumin, wherein betaine has a mass concentration ranging from 0.1 mg/mL to 20 mg/mL, and bovine serum albumin has a mass concentration ranging from 5 mg/mL to 100 mg/mL.

In one of the embodiments, the nucleic acid release agent further comprises proteinase K and lithium dodecyl sulfate, wherein proteinase K has a mass concentration ranging from 0.02 mg/mL to 1.5 mg/mL, and lithium dodecyl sulfate has a mass concentration ranging from 0.4 mg/mL to 30 mg/mL.

The present disclosure also provides a method for PCR amplification of a nucleic acid, comprising steps of mixing the nucleic acid release agent as described above with a sample, placing the mixture at 25°C to 60°C for 2 min to 10 min, and adding a PCR reaction solution for PCR amplification.

In one of the embodiments, the method further comprises a sample pretreatment step of mixing the sample with polyethylene glycol followed by centrifugation to collect a precipitate before mixing the nucleic acid release agent with the sample.

In one of the embodiments, the PCR amplification of intestinal viruses in the sample is performed at following conditions:
reverse transcription at 48°C to 52°C for 28 min to 32 min;
thermal denaturation at 93°C to 97°C for 0.9 min to 1.1 min;
several cycles of amplification at 93°C to 97°C for 13 sec to 17 sec followed by 53°C to 57°C for 28 sec to 32 sec;
cooling at 23°C to 27°C for 8 sec to 12 sec.

In one of the embodiments, the PCR amplification of hepatitis C virus in the sample is performed at following conditions:
pre-denaturation and enzyme activation at 93°C to 97°C for 0.9 min to 1.1 min;
reverse transcription at 58°C to 62°C for 28 min to 32 min;
thermal denaturation at 93°C to 97°C for 0.9 min to 1.1 min;
several cycles of amplification at 93°C to 97°C for 13 sec to 17 sec followed by 58°C to 62°C for 28 sec to 32 sec;
cooling at 23°C to 27°C for 8 sec to 12 sec.

In one of the embodiments, the PCR amplification of respiratory viruses in the sample is performed at following conditions:
reverse transcription at 48°C to 52°C for 28 min to 32 min;
thermal denaturation at 93°C to 97°C for 0.9 min to 1.1 min;
several cycles of amplification at 93°C to 97°C for 13 sec to 17 sec followed by 58°C to 62°C for 28 sec to 32 sec;
cooling at 23°C to 27°C for 8 sec to 12 sec.

In one of the embodiments, the PCR amplification of respiratory bacteria in the sample is performed at following conditions:
UDG enzyme reaction at 48°C to 52°C for 1.9 min to 2.1 min;
thermal denaturation at 92°C to 96°C for 2.9 min to 3.1 min;
several cycles of amplification at 92°C to 96°C for 8 sec to 12 sec followed by 58°C to 62°C for 18 sec to 22 sec;
extension and fluorescence collection at 73°C to 77°C for 18 sec to 22 sec;
melting curve: 62°C to 75°C.

The present disclosure also provides a PCR amplification kit, comprising the nucleic acid release agent as described above and a PCR reaction solution.

### BRIEF DESCRIPTION OF THE DRAWINGS

- FIG. 1: shows real-time fluorescence quantitative PCR amplification curves in Examples 1-25.
- FIG. 2: shows real-time fluorescence quantitative PCR amplification curves in Examples 26-50.
- FIG. 3: shows real-time fluorescence quantitative PCR amplification curves in Examples 51-70.
- FIG. 4: shows real-time fluorescence quantitative PCR amplification curves in Examples 71-90.

### DETAILED DESCRIPTION OF THE EMBODIMENTS

To make the present disclosure easy to understand, a more comprehensive description of the present disclosure will be given below, and better embodiments of the present disclosure are given below. However, the present disclosure can be implemented in many different forms and shall not be limited to the embodiments described herein. On the contrary, the purpose of providing these embodiments is to provide a more thorough and comprehensive understanding of the disclosure of the present disclosure.

Unless otherwise defined, all technical and scientific terms used herein have the same meanings as those generally understood by those skilled in the art of the present disclosure. The terms used in the specification of the present disclosure are only for the purpose of describing specific embodiments, rather than limiting the present disclosure. The term "and/or" as used herein includes any and all combinations of one or more related listed items.

In an embodiment of the present disclosure, a nucleic acid release agent comprises Tris-HCl, sodium chloride, potassium chloride, Tween 20, Triton X-100, ethyl phenyl polyethylene glycol and a strong base; wherein Tris-HCl has a molar concentration ranging from 0.5 mM to 500 mM, sodium chloride has a molar concentration from 20 mM to 500 mM, potassium chloride has a mass concentration ranging from 5 mg/mL to 8 mg/mL, Tween 20 has a volume percentage ranging from 0.1% to 2%, Triton X-100 has a volume percentage ranging from 0.1% to 3%, ethyl phenyl polyethylene glycol has a volume percentage ranging from 0.1% to 3%, and the strong base has a mass concentration ranging from 2 mg/mL to 50 mg/mL.

In order to achieve the effect of lysis, a lysis solution needs to be alkaline, but RNA is easily degraded in an alkaline environment. Moreover, a reverse transcriptase, which is required for PCR amplification and detection of RNA, is relatively fragile compared with the thermally stable TaqDNA polymerase and is easily inhibited in an alkaline environment, resulting in an inability to achieve the effect of reverse transcription. Therefore, RNA in the lysis solution generally cannot be directly amplified and detected by PCR. At present, pre-treating methods for PCR amplification and detection of RNA samples mainly include a boiling lysis method and a magnetic bead method. In the boiling lysis method, nucleic acids in a sample are released by boiling under the action of a lysis buffer and dissolved in the lysis buffer. While the cells are lysed in a boiling water bath, the proteins and chromosomes of the cells are denatured. Then the denatured proteins and other impurities are removed by centrifugation, and the nucleic acids in the supernatant are recovered for PCR amplification. However, coagulation of the proteins subjected to boiling at a high temperature causes part of the nucleic acids to be wrapped and run off with the centrifugation, leading directly to a decrease in the amount of template nucleic acids in the supernatant, which reduces the sensitivity of subsequent amplification and detection. In addition, aerosols are easily produced by boiling and heating, leading to sample contamination, which will result in false-positive results in subsequent detection. In the magnetic bead method, cells are lysed in a lysis solution, and free nucleic acid molecules are specifically adsorbed to the surface of the magnetic particles, while impurities such as proteins are not adsorbed but remained in the solution. After reacting for a certain period of time, the magnetic particles are separated from the liquid under the action of a magnetic field, followed by elution with an eluent to obtain pure nucleic acids. However, the magnetic bead method has complicated operation, requires a sample size that is generally 200 to 600 microliters, and has a very high demand on equipments, giving limitations on its promotion and use.

Based on the above mechanism, in the nucleic acid release agent of this embodiment, a certain proportion of the strong base lyses cells, which release nucleic acids. Sodium chloride and potassium chloride protect nucleic acids by coordinating the balance of intracellular and extracellular ions. Tris-HCl is for keeping a pH value stable during cell lysis and is better compatible with a PCR reaction solution in subsequent amplification. Triton X-100, on the one hand, can protect nucleic acids, especially single-stranded RNA, allowing RNA to be stored in an alkaline environment. On the other hand, Triton X-100 with potassium chloride in a certain ratio can reduce the inhibitory effect of the strong alkaline environment on the enzyme in the PCR reaction, thereby ensuring the amplification efficiency of RNA. Tween 20 and ethyl phenyl polyethylene glycol can protect reverse transcriptase, so that the reverse transcriptase works normally in the alkaline environment. Through the synergy of the above components, the nucleic acid release agent of this embodiment enables a sample containing RNA to directly release RNA from at room temperature, thereby avoiding the problem of sample contamination caused by aerosol generated by heating and effectively preventing RNA from degrading in the alkaline environment. More importantly, it can be directly mixed with the PCR reaction solution for PCR amplification to complete the amplification, without the need for complicated nucleic acid extraction and purification processes. This truly realizes a one-chamber, pollution-free, simple and rapid RNA amplification and detection, with high detection sensitivity and good repeatability. It can be understood that the nucleic acid release agent can be used not only for PCR amplification and detection of RNA samples, but also for multiple and combined amplification and detection of DNA samples or mixed samples of RNA and DNA.

Specifically, the strong base is sodium hydroxide or potassium hydroxide, and the like, which can be selected as required.

In a specific example, the nucleic acid release agent further comprises betaine and bovine serum albumin, wherein betaine has a mass concentration ranging from 0.1 mg/mL to 20 mg/mL, and bovine serum albumin has a mass concentration ranging from 5 mg/mL to 100 mg/mL. The certain proportion of betaine and bovine serum albumin added may collaborate with Triton X-100 to better protect RNA under alkaline conditions and prevent polymerase and reverse transcriptase from their denaturation, thereby guaranteeing rapid release and amplification of RNA to achieve rapid detection of RNA samples.

In a specific example, the nucleic acid release agent further comprises proteinase K and lithium dodecyl sulfate, wherein proteinase K has a mass concentration ranging from 0.02 mg/mL to 1.5 mg/mL, and lithium dodecyl sulfate has a mass concentration ranging from 0.4 mg/mL to 30 mg/mL. The certain proportion of proteinase K and lithium dodecyl sulfate added may denature and degrade RNase, thereby further protecting RNA from its degradation.

In an embodiment of the present disclosure, a method for PCR amplification of a nucleic acid comprises steps of: mixing the nucleic acid release agent as described above with a sample, placing the mixture at 25°C to 60°C for 2 min to 10 min, and adding a PCR reaction solution for PCR amplification.

The method for PCR amplification of the nucleic acid in this embodiment realizes the operation of direct amplification without extraction and purification for nucleic acid samples such as RNA, that is, the amplification and detection of the nucleic acid sample can be done by adding the nucleic acid release agent described above to the sample to release the nucleic acids from the cells, adding the PCR reaction solution and performing PCR amplification such as real-time fluorescent quantitative PCR directly, without the need for boiling and heating or extraction and purification processes, and the like. This truly realizes a one-chamber, pollution-free, simple and rapid RNA amplification and detection, with high detection sensitivity and good repeatability.

In a specific example, a volume ratio of the nucleic acid release agent to the sample is 1:1 to 1:5. Optionally, the samples can be of a variety of types including serum, plasma, an oropharyngeal swab, a nasopharyngeal swab, alveolar lavage fluid, stool and the like, and after being mixed with the nucleic acid release agent, can be directly used in detection methods downstream, such as PCR amplification or gene chip.

In a specific example, the PCR reaction solution comprises deoxyribonucleoside triphosphate, a forward primer, a reverse primer, DNA polymerase, reverse transcriptase and amplification buffer, and the like. It can be understood that, according to different types and purposes of the PCR reaction, the composition of the PCR reaction solution can be selected as needed and is not limited thereto. For example, when performing real-time fluorescent quantitative PCR, the PCR reaction solution also includes fluorescent probes or fluorescent dyes, etc.

In a specific example, the method further comprises a sample pretreatment step of mixing the sample with polyethylene glycol followed by centrifugation to collect a precipitate before mixing the nucleic acid release agent with the sample. Specifically, polyethylene glycol is PEG-6000, with a concentration of 0.5% to 5% by volume. Polyethylene glycol as a nucleic acid sedimentation agent can be used for the treatment of complex samples. It can effectively capture RNA viruses in free form and increase the sensitivity of later detection without affecting the PCR reaction system, and can significantly improve the performance of rapid PCR detection of RNA. For example, for cell preserving fluids containing high-salt solutions, virus preserving fluids, or hemolyzed blood samples, 50 to 5000 microliters of the sample can be taken and PEG solution in an equal volume can be added thereto followed by centrifugation at 3000 to 13000 rpm/min for 1 to 10 min, with the supernatant discarded and the pellet left, and then 50 to 100 microliters of nucleic acid release agent is added.

In a specific example, the PCR amplification of intestinal viruses in the sample is performed at following conditions:
reverse transcription at 48°C to 52°C for 28 min to 32 min;
thermal denaturation at 93°C to 97°C for 0.9 min to 1.1 min;
several cycles of amplification at 93°C to 97°C for 13 sec to 17 sec followed by 53°C to 57°C for 28 sec to 32 sec;
cooling at 23°C to 27°C for 8 sec to 12 sec.

In a specific example, the PCR amplification of hepatitis C virus (HCV) in the sample is performed at following conditions:
pre-denaturation and enzyme activation at 93°C to 97°C for 0.9 min to 1.1 min;
reverse transcription at 58°C to 62°C for 28 min to 32 min;
thermal denaturation at 93°C to 97°C for 0.9 min to 1.1 min;
several cycles of amplification at 93°C to 97°C for 13 sec to 17 sec followed by 58°C to 62°C for 28 sec to 32 sec;
cooling at 23°C to 27°C for 8 sec to 12 sec.

In a specific example, the PCR amplification of respiratory viruses in the sample is performed at following conditions:
reverse transcription at 48°C to 52°C for 28 min to 32 min;
thermal denaturation at 93°C to 97°C for 0.9 min to 1.1 min;
several cycles of amplification at 93°C to 97°C for 13 sec to 17 sec followed by 58°C to 62°C for 28 sec to 32 sec;
cooling at 23°C to 27°C for 8 sec to 12 sec.

In a specific example, the PCR amplification of respiratory bacteria in the sample is performed at following conditions:
UDG enzyme reaction at 48°C to 52°C for 1.9 min to 2.1 min;
thermal denaturation at 92°C to 96°C for 2.9 min to 3.1 min;
several cycles of amplification at 92°C to 96°C for 8 sec to 12 sec followed by
58°C to 62°C for 18 sec to 22 sec;
extension and fluorescence collection at 73°C to 77°C for 18 sec to 22 sec;
melting curve: 62°C to 75°C.

It can be understood that the conditions for PCR amplification are not limited to the above specific examples, and can be adjusted according to different PCR types and purposes.

In an embodiment of the present disclosure, a PCR amplification kit comprises the nucleic acid release agent as described above and a PCR reaction solution. The PCR amplification kit in this embodiment realizes the operation of direct amplification without extraction and purification for nucleic acid samples such as RNA, that is, the amplification and detection of the nucleic acid sample can be done by adding the nucleic acid release agent described above to the sample to release the nucleic acids from the cells, adding the PCR reaction solution and performing PCR amplification such as real-time fluorescent quantitative PCR directly, without the need for boiling and heating or extraction and purification processes, and the like. This truly realizes a one-chamber, pollution-free, simple and rapid RNA amplification and detection, with high detection sensitivity and good repeatability.

The following are specific examples.

### 1. Detection for intestinal viruses

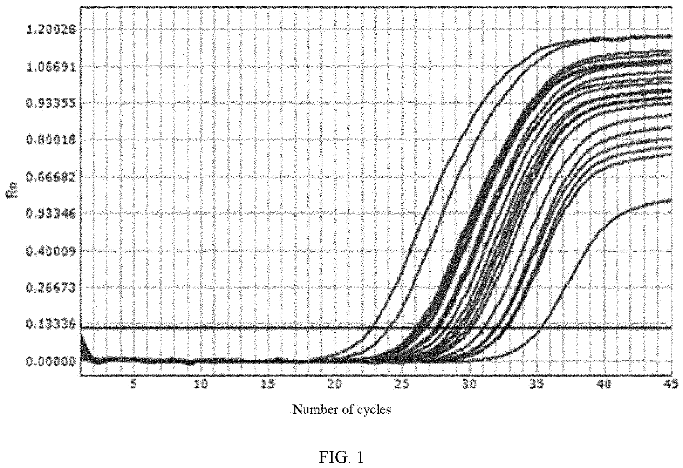

Examples 1 to 25: 25 intestinal virus throat swab samples (virus preserving fluid vehicle) were prepared. 100µL of each sample was taken and centrifugated at 12000rpm/min for 10min, with the supernatant discarded. After adding 50 µL of a nucleic acid release agent and standing for 10 min, 10 µL of the nucleic acid release agent-treated sample was mixed with 40 µL of a PCR reaction solution for performing real-time fluorescence quantitative PCR amplification. The amplification curve is shown in FIG. 1. The nucleic acid release agent used in Examples 1 to 25 comprised Tris-HCl, sodium chloride, potassium chloride, Tween 20, Triton X-100, ethyl phenyl polyethylene glycol, betaine, bovine serum albumin, Proteinase K, lithium dodecyl sulfate and sodium hydroxide; wherein Tris-HCl had a molar concentration of 0.5 mM, sodium chloride had a molar concentration of 500 mM, Tween 20 had a volume percentage of 0.1%, Triton X-100 had a volume percentage of 3%, ethyl phenyl polyethylene glycol had a volume percentage of 0.1%, potassium chloride had a mass concentration of 8 mg/mL, sodium hydroxide had a mass concentration of 2 mg/mL, betaine had a mass concentration of 20 mg/mL, bovine serum albumin had a mass concentration of 5 mg/mL, proteinase K had a mass concentration of 1.5 mg/mL, and lithium dodecyl sulfate had a mass concentration of 0.4 mg/mL.

Comparative Examples 1-25: The above 25 samples were treated by the magnetic bead method, and real-time fluorescent quantitative PCR amplification was performed. Comparative Examples 26-35 were basically the same as Examples 1 to 10, except that Tween 20 and ethyl phenyl polyethylene glycol were not comprised in the nucleic acid release agent.

Comparative Examples 36-45 were basically the same as Examples 11 to 20, except that Triton X-100 was not comprised in the nucleic acid release agent. Comparative Examples 46-50 were basically the same as Examples 21 to 25, except that Triton X-100 in the nucleic acid release agent had a volume percentage of 8%, and potassium chloride had a mass concentration of 15 mg/mL.

Ct values in the Examples and Comparative Examples are shown in Table 1. PCR amplification was performed at following conditions:
reverse transcription at 50°C for 30 min;
thermal denaturation at 95°C for 1 min;
45 cycles of amplification at 95°C for 15 sec followed by 55°C for 30 sec;
cooling at 25°C for 10 sec.

**Table 1**

| | Comparative Examples 1 to 13 | Examples 1 to 13 | | Comparative Examples 14 to 25 | Examples 14 to 25 |
|---|---|---|---|---|---|
| Sample 1 | 24.49 | 24.07 | Sample 14 | 20.06 | 21.68 |
| Sample 2 | 25.88 | 25.32 | Sample 15 | 24.21 | 20.64 |
| Sample 3 | 25.55 | 23.61 | Sample 16 | 23.30 | 23.63 |
| Sample 4 | 23.89 | 23.55 | Sample 17 | 26.39 | 27.63 |
| Sample 5 | 27.36 | 26.06 | Sample 18 | 29.66 | 28.03 |
| Sample 6 | 26.61 | 28.12 | Sample 19 | 24.24 | 21.44 |
| Sample 7 | 28.56 | 29.06 | Sample 20 | 34.01 | 34.23 |
| Sample 8 | 44.09 | 37.96 | Sample 21 | 28.69 | 27.57 |
| Sample 9 | 32.44 | 31.82 | Sample 22 | 27.10 | 25.31 |
| Sample 10 | 30.04 | 29.27 | Sample 23 | 29.63 | 31.11 |
| Sample 11 | 22.54 | 24.24 | Sample 24 | 31.99 | 32.50 |
| Sample 12 | 24.18 | 25.83 | Sample 25 | 27.69 | 27.60 |
| Sample 13 | 31.13 | 29.93 | | | |

According to the test results, samples that were positive for intestinal virus can be detected in all Comparative Examples 1 to 25 and Examples 1 to 25 with a consistent rate of results of 100% and good accuracy. However, the samples that were positive for intestinal virus cannot be stably and successfully detected in Comparative Examples 26-50. In addition, a smaller Ct value indicates higher detection sensitivity. Comparing the Ct values, it can be seen that in the examples using the nucleic acid release agent and the method for PCR amplification of the nucleic acid of the present disclosure, the sensitivity of the amplification and detection of RNA samples is equivalent to that using the magnetic bead method.

### 2. Combined detection for respiratory bacteria

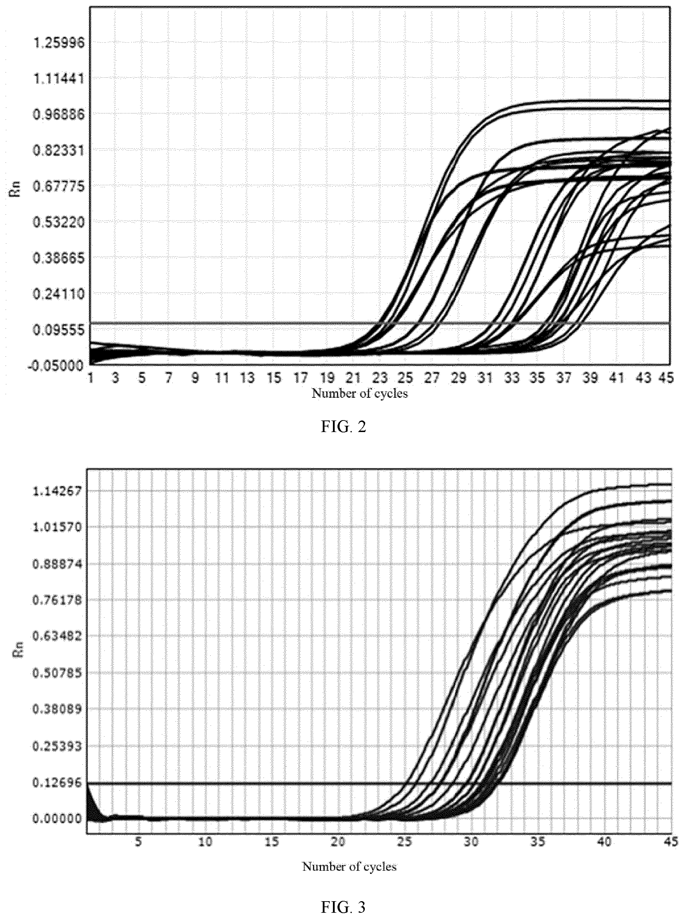

Examples 26 to 50: 25 respiratory tract sputum samples (normal saline vehicle) were prepared. 5 µL of each sample was taken, and 5 µL of a nucleic acid release agent was added thereto, followed by standing for 10 min. 40 µL of a PCR reaction solution was added with mixing for performing real-time fluorescence quantitative PCR amplification. The amplification curve is shown in FIG. 2. The nucleic acid release agents used comprised Tris-HCl, sodium chloride, potassium chloride, Tween 20, Triton X-100, ethyl phenyl polyethylene glycol, betaine, bovine serum albumin, proteinase K, lithium dodecyl sulfate and sodium hydroxide; wherein Tris-HCl had a molar concentration of 500 mM, sodium chloride had a molar concentration of 20 mM, Tween 20 had a volume percentage of 2%, Triton X-100 had a volume percentage of 0.1%, ethyl phenyl polyethylene glycol had a volume percentage of 3%, potassium chloride had a mass concentration of 5 mg/mL, sodium hydroxide had a mass concentration of 50 mg/mL, betaine had a mass concentration of 0.1 mg/mL, bovine serum albumin had a mass concentration of 100 mg/mL, proteinase K had a mass concentration of 0.02 mg/mL, and lithium dodecyl sulfate had a mass concentration of 30 mg/mL.

Comparative Examples 51-75: The above 25 samples were treated by the magnetic bead method, and real-time fluorescent quantitative PCR amplification was performed.

Ct values in the Examples and Comparative Examples are shown in Table 2. PCR amplification was performed at following conditions:
UDG enzyme reaction at 50°C for 2 min;
thermal denaturation at 94°C for 3 min;
45 cycles of amplification at 94°C for 10 sec followed by 60°C for 20 sec;
extension and fluorescence collection at 75°C for 20 sec;
melting curve: 62°C to 75°C.

**Table 2**

| | Comparative Examples 51 to 63 | Examples 26 to 38 | | Comparative Examples 64 to 75 | Examples 39 to 50 |
|---|---|---|---|---|---|
| Sample 1 | 35.36 | 31.34 | Sample 14 | 23.35 | 24.14 |
| Sample 2 | 29.47 | 24.54 | Sample 15 | 36.03 | 32.69 |
| Sample 3 | 30.18 | 31.1 | Sample 16 | 36.2 | 35.99 |
| Sample 4 | 37.23 | 36.56 | Sample 17 | 36.02 | 35.95 |
| Sample 5 | 33.21 | 28.44 | Sample 18 | 33.44 | 33.2 |
| Sample 6 | 27.94 | 25.69 | Sample 19 | 38.34 | 38.32 |
| Sample 7 | 26.58 | 24.945 | Sample 20 | 30.76 | 29.145 |
| Sample 8 | 31.19 | 30.495 | Sample 21 | 36.49 | 30.85 |
| Sample 9 | 33.64 | 29.675 | Sample 22 | 26.22 | 25.925 |
| Sample 10 | 37.95 | 34.455 | Sample 23 | 23.01 | 22.96 |
| Sample 11 | 32.68 | 24.13 | Sample 24 | 31.39 | 27.32 |
| Sample 12 | 27.58 | 23.355 | Sample 25 | 29.26 | 24.015 |
| Sample 13 | 34.14 | 32.605 | | | |

According to the test results, samples that were positive for respiratory bacteria can be detected in all comparative examples and examples with a consistent rate of results of 100% and good accuracy. In addition, a smaller Ct value indicates higher detection sensitivity. Comparing the Ct values, it can be seen that in the examples using the nucleic acid release agent and the method for PCR amplification of the nucleic acid of the present disclosure, the sensitivity of the multiple amplification and detection of bacteria was equivalent to that using the magnetic bead method. It also shows that the nucleic acid release agent and the method for PCR amplification of the nucleic acid of the present disclosure are not only applicable to amplification and detection of RNA samples, but also can be applied to amplification and detection of DNA samples.

### 3. Detection for HCV viruses

Examples 51 to 70: 20 HCV serum samples were prepared. 15 µL of each sample was taken, and 5 µL of a nucleic acid release agent was added thereto, followed by standing for 10 min. 30 µL of a PCR reaction solution was added with mixing for performing real-time fluorescence quantitative PCR amplification. The amplification curve is shown in FIG. 3. The nucleic acid release agent used comprised Tris-HCl, sodium chloride, potassium chloride, Tween 20, Triton X-100, ethyl phenyl polyethylene glycol, betaine, bovine serum albumin, and sodium hydroxide; wherein Tris-HCl had a molar concentration of 200 mM, sodium chloride had a molar concentration of 250 mM, Tween 20 had a volume percentage of 1%, Triton X-100 had a volume percentage of 2%, ethyl phenyl polyethylene glycol had a volume percentage of 2%, potassium chloride had a mass concentration of 7 mg/mL, sodium hydroxide had a mass concentration of 25 mg/mL, betaine had a mass concentration of 10 mg/mL, and bovine serum albumin had a mass concentration of 60 mg/mL.

Examples 91 to 110: the same HCV serum samples as in Examples 51 to 70 were used. 15 µL of each sample was taken, and 5 µL of a nucleic acid release agent was added thereto, followed by standing for 10 min. 30 µL of a PCR reaction solution was added with mixing for performing real-time fluorescence quantitative PCR amplification. The nucleic acid release agent is the same as the nucleic acid release agent used in Examples 1 to 25.

Ct values in the examples are shown in Table 3. PCR amplification was performed at following conditions:
pre-denaturation and enzyme activation at 95°C for 1 min;
reverse transcription at 60°C for 30 min;
thermal denaturation at 95°C for 1 min;
45 cycles of amplification at 95°C for 15 sec followed by 60°C for 30 sec;
cooling at 25°C for 10 sec.

**Table 3**

| | Examples 91 to 100 | Examples 51 to 60 | | Examples 101 to 110 | Examples 61 to 70 |
|---|---|---|---|---|---|
| Sample 1 | 30.25 | 31.32 | Sample 11 | 24.56 | 25.13 |
| Sample 2 | 27.66 | 29.78 | Sample 12 | 25.60 | 26.03 |
| Sample 3 | 28.31 | 30.28 | Sample 13 | 27.06 | 27.92 |
| Sample 4 | 29.00 | 31.23 | Sample 14 | 27.95 | 28.88 |
| Sample 5 | 28.63 | 31.81 | Sample 15 | 31.05 | 31.64 |
| Sample 6 | 27.25 | 27.94 | Sample 16 | 28.60 | 30.55 |
| Sample 7 | 27.21 | 26.85 | Sample 17 | 29.67 | 30.76 |
| Sample 8 | 29.29 | 31.27 | Sample 18 | 30.44 | 31.49 |
| Sample 9 | 28.63 | 31.64 | Sample 19 | 19.78 | 20.25 |
| Sample 10 | 30.55 | 31.95 | Sample 20 | 33.20 | 32.01 |

According to FIG. 3, it can be seen that in samples that were positive for HCV can be detected in all examples using the nucleic acid release agent and the method for PCR amplification of the nucleic acid of the present disclosure with good accuracy. In addition, it can be seen from Table 3 that the detection sensitivity in Examples 51 to 70 is slightly worse than that in Examples 91 to 100, indicating that the effect of the nucleic acid release agent used in Examples 51 to 70 is slightly inferior to that of the nucleic acid release agent used in Examples 91 to 100.

### 4. Detection for respiratory viruses

Examples 71 to 90: 20 respiratory virus throat swab samples (normal saline vehicle) were prepared. 100 µL of each sample was taken and centrifugated at 12000 rpm/min for 10 min, with the supernatant discarded. After adding 50 µL of a nucleic acid release agent and standing for 10 min, 10 µL of the nucleic acid release agent-treated sample was mixed with 40 µL of a PCR reaction solution for performing real-time fluorescence quantitative PCR amplification. The amplification curve is shown in FIG. 1. The nucleic acid release agent used comprised Tris-HCl, sodium chloride, potassium chloride, Tween 20, Triton X-100, ethyl phenyl polyethylene glycol, and sodium hydroxide; wherein Tris-HCl had a molar concentration of 400 mM, sodium chloride had a molar concentration of 150 mM, Tween 20 had a volume percentage of 0.8%, Triton X-100 had a volume percentage of 1.2%, ethyl phenyl polyethylene glycol had a volume percentage of 1.5%, potassium chloride had a mass concentration of 6 mg/mL, and sodium hydroxide had a mass concentration of 15 mg/mL.

Examples 111 to 130: the same respiratory virus throat swab samples as in Examples 71 to 90 were used. 100 µL of each sample was taken and centrifugated at 12000 rpm/min for 10min, with the supernatant discarded. After adding 50 µL of a nucleic acid release agent and standing for 10 min, 10 µL of the nucleic acid release agent-treated sample was mixed with 40 µL of a PCR reaction solution for performing real-time fluorescence quantitative PCR amplification. The nucleic acid release agent was the same as the nucleic acid release agent used in Examples 51 to 70.

Ct values in the examples are shown in Table 4. PCR amplification was performed at following conditions:
reverse transcription at 50°C for 30 min;
thermal denaturation at 95°C for 1 min;
45 cycles of amplification at 95°C for 15 sec followed by 60°C for 30 sec;
cooling at 25°C for 10 sec.

**Table 4**

| | Examples 111 to 120 | Examples 71 to 80 | | Examples 121 to 130 | Examples 81 to 90 |
|---|---|---|---|---|---|
| Sample 1 | 23.64 | 26.05 | Sample 11 | 27.56 | 28.69 |
| Sample 2 | 25.97 | 28.77 | Sample 12 | 28.67 | 29.61 |
| Sample 3 | 29.55 | 31.28 | Sample 13 | 26.22 | 28.32 |
| Sample 4 | 30.52 | 32.65 | Sample 14 | 27.88 | 29.78 |
| Sample 5 | 28.10 | 28.55 | Sample 15 | 31.36 | 31.54 |
| Sample 6 | 31.69 | 34.12 | Sample 16 | 30.69 | 33.69 |
| Sample 7 | 25.19 | 26.12 | Sample 17 | 29.89 | 29.47 |
| Sample 8 | 30.26 | 32.36 | Sample 18 | 30.79 | 32.34 |
| Sample 9 | 28.96 | 31.59 | Sample 19 | 29.20 | 32.69 |
| Sample 10 | 31.60 | 31.35 | Sample 20 | 33.59 | 33.06 |

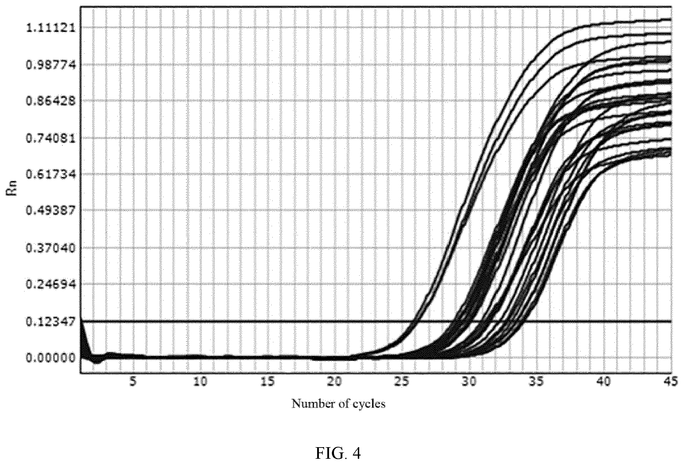

According to FIG. 4, it can be seen that samples that were positive for respiratory virus can be detected in all examples using the nucleic acid release agent and the method for PCR amplification of the nucleic acid of the present disclosure with good accuracy. In addition, it can be seen from Table 4 that the detection sensitivity in Examples 71 to 90 was slightly worse than that in Examples 111 to 130, indicating that the effect of the nucleic acid release agent used in Examples 71 to 90 was slightly inferior to that of the nucleic acid release agent used in Examples 111 to 130.

The technical features of the above-described embodiments may be combined arbitrarily. To simplify the description, not all of the possible combinations of the technical features in the above embodiments are described. However, all of the combinations of these technical features should be considered as within the scope of the present disclosure, as long as such combinations do not contradict with each other.

The above-described embodiments merely represent several embodiments of the present disclosure, and the description thereof is more specific and detailed, but it should not be construed as limiting the scope of the present disclosure. Therefore, the scope of the present disclosure shall be defined by the appended claims.

## Claims

1. A nucleic acid release agent, comprising Tris-HCl, sodium chloride, potassium chloride, Tween 20, Triton X-100, ethyl phenyl polyethylene glycol and a strong base; wherein Tris-HCl has a molar concentration ranging from 0.5 mM to 500 mM, sodium chloride has a molar concentration from 20 mM to 500 mM, potassium chloride has a mass concentration ranging from 5mg/mL to 8mg/mL, Tween 20 has a volume percentage ranging from 0.1% to 2%, Triton X-100 has a volume percentage ranging from 0.1% to 3%, ethyl phenyl polyethylene glycol has a volume percentage ranging from 0.1% to 3%, and the strong base has a mass concentration ranging from 2 mg/mL to 50 mg/mL.

2. The nucleic acid release agent according to claim 1, further comprising betaine and bovine serum albumin, wherein betaine has a mass concentration ranging from 0.1 mg/mL to 20 mg/mL, and bovine serum albumin has a mass concentration ranging from 5 mg/mL to 100 mg/mL.

3. The nucleic acid release agent according to claim 1, further comprising proteinase K and lithium dodecyl sulfate, wherein proteinase K has a mass concentration ranging from 0.02 mg/mL to 1.5 mg/mL, and lithium dodecyl sulfate has a mass concentration ranging from 0.4 mg/mL to 30 mg/mL.

4. A method for PCR amplification of a nucleic acid, comprising steps of: mixing the nucleic acid release agent according to any one of claims 1 to 3 with a sample, placing the mixture at 25°C to 60°C for 2 min to 10 min, and adding a PCR reaction solution for PCR amplification.

5. The method according to claim 4, further comprising a sample pretreatment step of mixing the sample with polyethylene glycol followed by centrifugation to collect a precipitate before mixing the nucleic acid release agent with the sample.

6. The method according to claim 4, wherein the PCR amplification of intestinal viruses in the sample is performed at following conditions:
reverse transcription at 48°C to 52°C for 28 min to 32 min;
thermal denaturation at 93°C to 97°C for 0.9 min to 1.1 min;
several cycles of amplification at 93°C to 97°C for 13 sec to 17 sec followed by 53°C to 57°C for 28 sec to 32 sec;
cooling at 23°C to 27°C for 8 sec to 12 sec.

7. The method according to claim 4, wherein the PCR amplification of hepatitis C virus in the sample is performed at following conditions:
pre-denaturation and enzyme activation at 93°C to 97°C for 0.9 min to 1.1 min;
reverse transcription at 58°C to 62°C for 28 min to 32 min;
thermal denaturation at 93°C to 97°C for 0.9 min to 1.1 min;
several cycles of amplification at 93°C to 97°C for 13 sec to 17 sec followed by 58°C to 62°C for 28 sec to 32 sec;
cooling at 23°C to 27°C for 8 sec to 12 sec.

8. The method according to claim 4, wherein the PCR amplification of respiratory viruses in the sample is performed at following conditions:
reverse transcription at 48°C to 52°C for 28 min to 32 min;
thermal denaturation at 93°C to 97°C for 0.9 min to 1.1 min;
several cycles of amplification at 93°C to 97°C for 13 sec to 17 sec followed by 58°C to 62°C for 28 sec to 32 sec;
cooling at 23°C to 27°C for 8 sec to 12 sec.

9. The method according to claim 4, wherein the PCR amplification of respiratory bacteria in the sample is performed at following conditions:
UDG enzyme reaction at 48°C to 52°C for 1.9 min to 2.1 min;
thermal denaturation at 92°C to 96°C for 2.9 min to 3.1 min;
several cycles of amplification at 92°C to 96°C for 8 sec to 12 sec followed by 58°C to 62°C for 18 sec to 22 sec;
extension and fluorescence collection at 73°C to 77°C for 18 sec to 22 sec;
melting curve: 62°C to 75°C.

10. A PCR amplification kit, comprising the nucleic acid release agent according to any one of claims 1 to 3 and a PCR reaction solution.

## Patentansprüche

1. Nukleinsäuretrennmittel mit Tris-HCl, Natriumchlorid, Kaliumchlorid, Tween 20, Triton X-100, Ethylphenylpolyethylenglykol und einer starken Base; wobei Tris-HCl eine molare Konzentration im Bereich von 0,5 mM bis 500 mM hat, Natriumchlorid eine molare Konzentration von 20 mM bis 500 mM hat, Kaliumchlorid eine Massenkonzentration im Bereich von 5 mg/mL bis 8 mg/mL hat, Tween 20 einen Volumenanteil im Bereich von 0,1 % bis 2 % hat, Triton X-100 einen Volumenanteil im Bereich von 0,1 % bis 3 % hat, Ethylphenylpolyethylenglykol einen Volumenanteil im Bereich von 0,1 % bis 3 % hat und die starke Base eine Massenkonzentration im Bereich von 2 mg/mL bis 50 mg/mL hat.

2. Nukleinsäuretrennmittel nach Anspruch 1, das ferner Betain und Rinderserumalbumin aufweist, wobei Betain eine Massenkonzentration im Bereich von 0,1 mg/mL bis 20 mg/mL hat und das Rinderserumalbumin eine Massenkonzentration im Bereich von 5 mg/mL bis 100 mg/mL hat.

3. Nukleinsäuretrennmittel nach Anspruch 1, das ferner Proteinase K und Lithiumdodecylsulfat aufweist, wobei Proteinase K eine Massenkonzentration im Bereich von 0,02 mg/mL bis 1,5 mg/mL hat und Lithiumdodecylsulfat eine Massenkonzentration im Bereich von 0,4 mg/mL bis 30 mg/mL hat.

4. Verfahren zur PCR-Amplifikation einer Nukleinsäure mit den Schritten: Mischen des Nukleinsäuretrennmittels nach einem der Ansprüche 1 bis 3 mit einer Probe, Halten der Mischung für 2 Minuten bis 10 Minuten bei 25 °C bis 60 °C und Hinzufügen einer PCR-Reaktionslösung für eine PCR-Amplifikation.

5. Verfahren nach Anspruch 4, das ferner einen Probenvorbehandlungsschritt zum Mischen der Probe mit Polyethylenglykol mit nachfolgender Zentrifugation zur Ansammlung einer Ausfällung vor dem Mischen des Nukleinsäuretrennmittels mit der Probe umfasst.

6. Verfahren nach Anspruch 4, wobei die PCR-Amplifikation von Darmviren in der Probe unter folgenden Bedingungen ausgeführt wird:
Reverstranskription bei 48 °C bis 52 °C für 28 Minuten bis 32 Minuten;
thermische Denaturierung bei 93 °C bis 97 °C für 0,9 Minuten bis 1,1 Minuten;
mehrere Amplifikationszyklen bei 93 °C bis 97 °C für 13 Sekunden bis 17 Sekunden und anschließend bei 53 °C bis 57 °C für 28 Sekunden bis 32 Sekunden;
Abkühlen bei 23 °C bis 27 °C für 8 Sekunden bis 12 Sekunden.

7. Verfahren nach Anspruch 4, wobei die PCR-Amplifikation von Hepatitis-C-Viren in der Probe unter folgenden Bedingungen ausgeführt wird:
Vor-Denaturierung und Enzymaktivierung bei 93 °C bis 97 °C für 0,9 Minuten bis 1,1 Minuten;
Reverstranskription bei 58 °C bis 62 °C für 28 Minuten bis 32 Minuten;
thermische Denaturierung bei 93 °C bis 97 °C für 0,9 Minuten bis 1,1 Minuten;
mehrere Amplifikationszyklen bei 93 °C bis 97 °C für 13 Sekunden bis 17 Sekunden und anschließend bei 58 °C bis 62 °C für 28 Sekunden bis 32 Sekunden;
Abkühlen bei 23 °C bis 27 °C für 8 Sekunden bis 12 Sekunden.

8. Verfahren nach Anspruch 4, wobei die PCR-Amplifikation von Atemwegsviren in der Probe unter folgenden Bedingungen ausgeführt wird:
Reverstranskription bei 48 °C bis 52 °C für 28 Minuten bis 32 Minuten;
thermische Denaturierung bei 93 °C bis 97 °C für 0,9 Minuten bis 1,1 Minuten;
mehrere Amplifikationszyklen bei 93 °C bis 97 °C für 13 Sekunden bis 17 Sekunden und anschließend bei 58 °C bis 62 °C für 28 Sekunden bis 32 Sekunden;
Abkühlen bei 23 °C bis 27 °C für 8 Sekunden bis 12 Sekunden.

9. Verfahren nach Anspruch 4, wobei die PCR-Amplifikation von Atemwegsbakterien in der Probe unter folgenden Bedingungen ausgeführt wird:
UDG-Enzymreaktion bei 48 °C bis 52 °C für 1,9 Minuten bis 2,1 Minuten;
thermische Denaturierung bei 92 °C bis 96 °C für 2,9 Minuten bis 3,1 Minuten;
mehrere Amplifikationszyklen bei 92 °C bis 96 °C für 8 Sekunden bis 12 Sekunden und anschließend bei 58 °C bis 62 °C für 18 Sekunden bis 22 Sekunden;
Extension und Fluoreszenzsammlung bei 73°C bis 77 °C für 18 Sekunden bis 22 Sekunden;
Schmelzkurve: 62 °C bis 75 °C.

10. PCR-Amplifikationskit mit dem Nukleinsäuretrennmittel nach einem der Ansprüche 1 bis 3 und einer PCR-Reaktionslösung.

## Revendications

1. Agent de libération d'acide nucléique, comprenant Tris-HCl, chlorure de sodium, chlorure de potassium, Tween 20, Triton X-100, éthylphénylpolyéthylène glycol et une base forte ; dans lequel le Tris-HCl a une concentration molaire allant de 0,5 mM à 500 mM, le chlorure de sodium a une concentration molaire de 20 mM à 500 mM, le chlorure de potassium a une concentration massique allant de 5 mg/mL à 8 mg/mL, le Tween 20 a un pourcentage volumique allant de 0,1 % à 2 %, le Triton X-100 a un pourcentage volumique allant de 0,1 % à 3 %, l'éthylphénylpolyéthylène glycol a un pourcentage volumique allant de 0,1 % à 3 %, et la base forte a une concentration massique allant de 2 mg/mL à 50 mg/mL.

2. Agent de libération d'acide nucléique selon la revendication 1, comprenant en outre de la bétaïne et de l'albumine de sérum bovin, la bétaïne ayant une concentration massique allant de 0,1 mg/mL à 20 mg/mL, et l'albumine de sérum bovin ayant une concentration massique allant de 5 mg/mL à 100 mg/mL.

3. Agent de libération d'acide nucléique selon la revendication 1, comprenant en outre de la protéinase K et du dodécylsulfate de lithium, la protéinase K ayant une concentration massique allant de 0,02 mg/mL à 1,5 mg/mL, et le dodécylsulfate de lithium ayant une concentration massique allant de 0,4 mg/mL à 30 mg/mL.

4. Procédé d'amplification PCR d'un acide nucléique, comprenant des étapes consistant : à mélanger l'agent de libération d'acide nucléique selon l'une quelconque des revendications 1 à 3 avec un échantillon, à placer le mélange à une température de 25 °C à 60 °C pendant 2 minutes à 10 minutes, et à ajouter une solution de réaction PCR pour l'amplification PCR.

5. Procédé selon la revendication 4, comprenant en outre une étape de pré-traitement d'échantillon consistant à mélanger l'échantillon avec du polyéthylène glycol suivie d'une centrifugation pour recueillir un précipité avant de mélanger l'agent de libération d'acide nucléique avec l'échantillon.

6. Procédé selon la revendication 4, dans lequel l'amplification PCR de virus intestinaux dans l'échantillon est effectuée dans les conditions suivantes :
transcription inverse à une température de 48 °C à 52 °C pendant 28 minutes à 32 minutes ;
dénaturation thermique à une température de 93 °C à 97 °C pendant 0,9 minute à 1,1 minute ;
plusieurs cycles d'amplification à une température de 93 °C à 97 °C pendant 13 secondes à 17 secondes puis de 53 °C à 57 °C pendant 28 secondes à 32 secondes ;
refroidissement à une température de 23 °C à 27 °C pendant 8 secondes à 12 secondes.

7. Procédé selon la revendication 4, dans lequel l'amplification PCR du virus de l'hépatite C dans l'échantillon est effectuée dans les conditions suivantes :
pré-dénaturation et activation enzymatique à une température de 93 °C à 97 °C pendant 0,9 minute à 1,1 minute ;
transcription inverse à une température de 58 °C à 62 °C pendant 28 minutes à 32 minutes ;
dénaturation thermique à une température de 93 °C à 97 °C pendant 0,9 minute à 1,1 minute ;
plusieurs cycles d'amplification à une température de 93 °C à 97 °C pendant 13 secondes à 17 secondes puis de 58 °C à 62 °C pendant 28 secondes à 32 secondes ;
refroidissement à une température de 23 °C à 27 °C pendant 8 secondes à 12 secondes.

8. Procédé selon la revendication 4, dans lequel l'amplification PCR de virus respiratoires dans l'échantillon est effectuée dans les conditions suivantes :
transcription inverse à une température de 48 °C à 52 °C pendant 28 minutes à 32 minutes ;
dénaturation thermique à une température de 93 °C à 97 °C pendant 0,9 minute à 1,1 minute,
plusieurs cycles d'amplification à une température de 93 °C à 97 °C pendant 13 secondes à 17 secondes puis de 58 °C à 62 °C pendant 28 secondes à 32 secondes ;
refroidissement à une température de 23 °C à 27 °C pendant 8 secondes à 12 secondes.

9. Procédé selon la revendication 4, dans lequel l'amplification PCR de bactéries respiratoires dans l'échantillon est effectuée dans les conditions suivantes :
réaction enzymatique UDG à une température de 48 °C à 52 °C pendant 1,9 minute à 2,1 minutes ;
dénaturation thermique à une température de 92 °C à 96 °C pendant 2,9 minutes à 3,1 minutes ;
plusieurs cycles d'amplification à une température de 92 °C à 96 °C pendant 8 secondes à 12 secondes puis de 58 °C à 62 °C pendant 18 secondes à 22 secondes ;
extension et recueil de fluorescence à une température de 73 °C à 77 °C pendant 18 secondes à 22 secondes ;
courbe de fusion : de 62 °C à 75 °C.

10. Trousse d'amplification PCR, comprenant l'agent de libération d'acide nucléique selon l'une quelconque des revendications 1 à 3 et une solution de réaction PCR.
